# EUROPEAN PATENT SPECIFICATION

(11) **EP 1 928 466 B1**
(45) Date of publication and mention of the grant of the patent: **22.11.2017**
(21) Application number: 06813236.4
(22) Date of filing: 27.07.2006
(51) Int. Cl.: A61K 8/49, A61Q 19/06, A61K 31/409, A61K 31/555

(54) **PDT TREATMENT METHOD FOR CELLULITE AND COSMETIC USE**
PDT-BEHANDLUNGSMETHODE GEGEN CELLULITE UND KOSMETISCHE VERWENDUNG
METHODE DE TRAITEMENT ANTI-CELLULITE PAR TPD ET APPLICATIONS COSMETIQUES

(30) Priority: 02.08.2005 US 704797 P; 20.07.2006 US 489873
(43) Date of publication of application: 11.06.2008
(73) Proprietor: biolitec Unternehmensbeteiligungs II AG, 1030 Vienna (AT)
(72) Inventor: CASTRO, Danilo, Suarez, Montevideo, 11300 (UY); NEUBERGER, Wolfgang, Labuan, 8700 F.T. (MY)
(74) Representative: Herrmann, Franz
(86) International application number: PCT/US2006/029284
(87) International publication number: WO 2007/016287

(56) References cited:
- WO-A1-99/48474
- WO-A1-2005/023220
- WO-A2-03/039478
- WO-A2-2007/084468
- DE-A1- 10 107 575
- US-A- 5 484 778
- US-A- 5 587 396
- US-A1- 2002 156 062
- US-A1- 2003 023 283
- US-A1- 2005 085 455

## Description

### Background of the Invention

### Field of the Invention

The present invention relates to the field of cosmetic treatment and, in particular, relates to a photodynamic therapy treatment to remove cellulite tissue and also excess adipose cells in areas of the body prone to buildup of such tissues.

### Invention Disclosure Statement

It is a well known fact that modern society has created an abundance of readily available foods, i.e., "fast foods," and also has created an environment where entertainment has fostered a sedentary life style of, for example, watching television, playing video games and talking on the phone while eating high caloric snack foods. This has allowed people to gain excessive weight by an increase in adipose tissue, fat cells. Certain heredity conditions further have also created areas of excessive fat cells that are difficult to remove in that they are in areas that are not affected or minimally affected by diet and exercise. In an area where fat cells have excessively accumulated, normally in the buttocks, hips , and thighs, especially in women, the collagen fibers are deformed which allows pockets of fat cells to build up and deform the skin surface into producing bubbles or ripples, also called cellulite.

"Edematous-fibrosclerotic panniculopathy" is a medical term used to describe cellulite. Cellulite affects 80-90% of women in their post-pubertal period. Cellulites are found commonly on the hips, thighs, and buttocks giving a dimpled appearance in those areas of the body. It is not a disorder but an issue of cosmetic concern to the individual. Cellulite is most often seen in women than in men due to the structural differences of their adipose tissue. Cellulite is not related to obesity or overweight, since it can occur even in normal and thin women.

Cellulite is different from fat cell layer in the body. Most fatty deposit in the individual depends on his/her weight, life style and genetic makeup. Fat layer in the body had important function of insulation, protecting vital organs etc. while cellulite is largely due to structural conformation below the skin which appears as lumpy pockets of trapped fat giving uneven dimpling or orange peel skin.

Cellulite develops in the hypodermis or subcutaneous fat layer, where fat lobes are organized into chambers by surrounding strands of connective tissue. Below this layer is scarpus fascia in which fat cells get larger when weight is gained. This layer is divided into chambers by connective tissue, which attaches the top layer of the skin to the lower layers of muscle. When these connective tissues become weak, the scarpus fascia bulges upward, causing the characteristic uneven, dimpled appearance in the skin.

Most procedures are ineffective in removing cellulite except for a long term dieting and exercise. The development of cellulite is genetically driven and is considered a normal condition and is thus difficult to remove.

Excessive fat depositions or "lipodystrophies" are produced by a disproportionate increase in the deeper section of the subcutaneous cellular tissues of fat cells.

Lipodystrophies are produced because the adiposities have a hereditary genetic code which makes them evolve in a specific way. Each adipocite cell has Beta 1 (lipogenetic) and Alfa 2 (lipolitic) receptors in its membrane. When there are more Beta 1 receptors on a particular area, then a localized obesity or lipodystrophy is produced. Because of the excessive Beta receptors in certain families, these families have a tendency for enlarged legs, breasts, waists, etc. On these patients treatment with low caloric diets exclusively is normally not successful which leads to abandonment of the treatment and recovering whatever localized fat was removed and returning to the same unhealthful practices.

The only effective way to treat cellulite is to directly act on the genetically altered fat tissues and similar tissues in the area of treatment.

Historically, different methods have been developed to treat this problem and billions of dollars are being spent annually by people to remove or reduce fat tissue in these areas of the body. By the late 70's liposuction started to be used followed by liposculpture in the late 80's being an improved liposuction performed under local anesthesia using traumatic trocars to remove fluids. Later ultrasonic liposculpture was developed by mid 90's and there are some reports on "laser liposuction" (using an external laser source) but laser assisted liposuction has not been clearly proved to be effective so far.

Among the patents covering the state of the art are the following.

U.S. Pat. No. 6,206,873 by Paolini, et al., titled, "Device and Method for Eliminating Adipose Layers by Means of Laser Energy," discloses a hollow needle with an optical fiber in the center. The fat tissue, adipose cells, is liquidized when the cell walls are broken. The fluid is removed by suction through the needle. The laser is used to simply thermally degrade the cell walls. Laser wavelength range is noted as from 0.75 to 2.5 microns but a preferred wavelength of 1.06 is called out. A rounded optical fiber end is shown in Fig. 3 beyond the needle end. Paolini et al. use a Nd:YAG type of laser and note a wavelength range above. Paolini et al. further note that the liquid produced may be removed from the body by normal absorption.

While in U.S. Pat. No.6,605,080 Altshuler et al. disclose the removal of lipid rich tissue using external laser, a YAG source as well as other lasers whose output energy are in a wavelength range of 880 to 935 nm, 1150 to 1230 nm or 2280 to 2360 nm. It is noted that the radiation in the lower bands, specifically, 900 to 930, and 1150 to 1230, are preferred in the treatment of fat tissue. Specifically, wavelength regions near water/OH absorptions are identified as not preferred. Also they recommend the use of a cooling system..

U.S. Pat. No. 6,743,215 by Bernakei, titled, "Method and Apparatus for Skin Absorption Enhancement and Cellulite Reduction" discloses a process including the application of a compound upon an abraded skin surface followed by electrical and mechanical to remove cellulite.

Publication WO 99/48474 by A. Casale, entitled, "Pharmaceutical or Cosmetic Compositions Containing Photosensitizer Substances," discloses a photosensitizer formulation of liposomes with the PS activated by a light of the wavelength between 700 and 900 nm. The light is absorbed by the photosensitizer to raise the temperature causing the liposome to melt and to release an active ingredient.

DE 101 07 575 A1 discloses a non-invasive reduction of fatty tissue by the use of a photodynamic effect, which induces apoptosis to destroy the fatty cells.

Most of the prior art methods mentioned above are useful for fat reduction (over weight or obesity), while cellulite is a different condition related to fat cells which cannot be addressed effectively using the above methods. Presently there is no truly effective treatment for cellulite.

There is thus a need for treatment techniques that minimizes surface distortion, post operative complications, and removes or reduces cellulite problem from selected areas. The present invention satisfies that need.

### Objectives and Brief Summary of the Invention

It is an objective of the present invention to provide a method of cosmetic treatment using laser, LED or other (e.g. a filtered lamp) radiation in conjunction with a photo-drug for the destruction of cellulite tissue in the area of treatment.

It is another objective of the present invention to provide a method of cosmetic treatment using radiation and a photo-drug that directly affects the sub-dermal fatty tissues.

It is yet another objective of the present invention to provide a method of cosmetic treatment using PhotoDynamic Therapy (PDT) to directly affect adiposites causing emulsification of the fatty tissue and then elimination by absorption, removal by the lymphatic system and by drainage.

It is still another objective of the present invention to provide a method of cosmetic treatment using PDT to directly affect the fatty tissues without destruction of the structural tissues.

It is a further objective of the present invention to provide a photosensitizer mixture to be used in a method of cosmetic treatment using PDT to reduce cellulites.

Briefly stated, the present invention provides a photosensitizer mixture and a method of treating cellulites by means of a percutaneous application of the mixture into the area of cellulite buildup followed by light illumination is presented. The photosensitizer can be combined with one or more cellular products including adipose cells and/or collagen or hyaluronic acid or compounds that have been previously removed by liposuction. This mixture can also include other compounds such as Lipofundin MCT 10 % to improve the photosensitizer's diffusion or to dilute it Varying concentrations are used depending on the area of treatment as well as the stage of the cellulites and whether the cellulites present a depressed area in the skin or an elevated area. The cosmetic treatment method substantially reduces or removes localized lipodystrophies and/or flaccidity and/or cellulite by localized laser, LED or other light irradiation of the area of treatment having a photosensitizer applied therein. The light energy is applied to destroy the "fat" cells by a combination of chemical reactions, primarily, and temperature wherein the cell walls break releasing the cell fluid. The light radiation is generally applied through devices to guide the radiation to the area of treatment. One or more light sources such as laser diodes or LEDs may be coupled into one or more optical fibers to increase the area of coverage as well as increase the amount of radiation in that area of coverage. Optical fibers can be introduced percutaneously or possibly interstitially into the area of treatment. Cell fluid in the area of treatment is removed by a combination of techniques. Quick and lasting cosmetic changes in areas having prior untreatable cellulite fat tissues are achieved while minimizing trauma.

The above, and other objects, features and advantages of the present invention will become apparent from the following description read in conjunction with the accompanying drawings.

### Brief Description of Figures

Figures 1A and 1B illustrate the before and after treatment of a hip area within days after treatment of a first patient by the method/composition of the present invention;
Figures 2A and 2B illustrate the before and after treatment of a lower buttock area within days after treatment of said first patient by the method/composition of the present invention;
Figures 3A and 3B illustrate the before and after treatment of a thigh area within days after treatment of said first patient by the present invention;
Figures 4A, and 4B illustrate a second patient having cellulites in typical areas of the body;
Figures 5A and 5B illustrate the second patient, after having treatment by the present invention, showing the absence of cellulites in these same typical areas.
Figures 6 illustrate a third patient before treatment and then 1 month and 6 months after treatment by the method and composition of the present invention;
Figure 7 illustrates the process of Temoporfin interacting with cells.
Figure 8 illustrates the chemical makeup of Temoporfin.
Figures 9A and 9B illustrate appropriate marking of sectors having cellulite thereon.
Figure 10 presents before and after example pictures, from a group of photos in the USA application of varying days, for 8 female patients, ranging in age from 23 to 56 years initially having Stage II,III or IV cellulites. It pictorially demonstrates the success of the present invention in reducing the stage of cellulites in patients.

### Detailed Description of Preferred Embodiments

Many women suffer, have suffered or are going to be at some time afflicted with cellulites in their life. It is an esthetic pathology that only has pathological general connotations.

It is a problem that belongs to mostly women, in general, and normally women older than 20 years.

The appearance of cellulites is a psychologically depressing event to many women because it appears in body areas that are exposed when wearing bathing suits, for example. The skin has a bubbly appearance with depressions and hills. Millions of dollars are spent by women seeking a cure each year for numerous advertised remedies by the cosmetology industry. Many of these remedies do not provide any long term solutions but only temporary relief by the elimination of excess water from cellular tissues.

There is not an adequate definitive solution, just medical and cosmetologic treatments that generally do not achieve the patient's complete satisfaction.

The goals of the present invention in regards to the treatment of cellulites is to: (1) provide a method of esthetic alterations of the skin for improving its visual aspect; (2) provide a process for homeostasis of the cells that are altered in the treatment of cellulites; and (3) increase of the web of collagen for optimizing the results of the adipose graft when treating cellulites.

In the present invention, suitable photosensitizers porphyrins and their derivatives, including temoporfin, chlorins, bacteriopheophorbide, bacteriochlorins etc are used. The selected photosensitizer can be administered using suitable delivery systems like liposomes, prodrug etc for efficient drug delivery to selected target cell. As commonly used throughout this specification and claims 'photosensitizer' is used to include precursors of photosensitizers, which naturally become photosensitizers after introduction into a patient as the precursor.

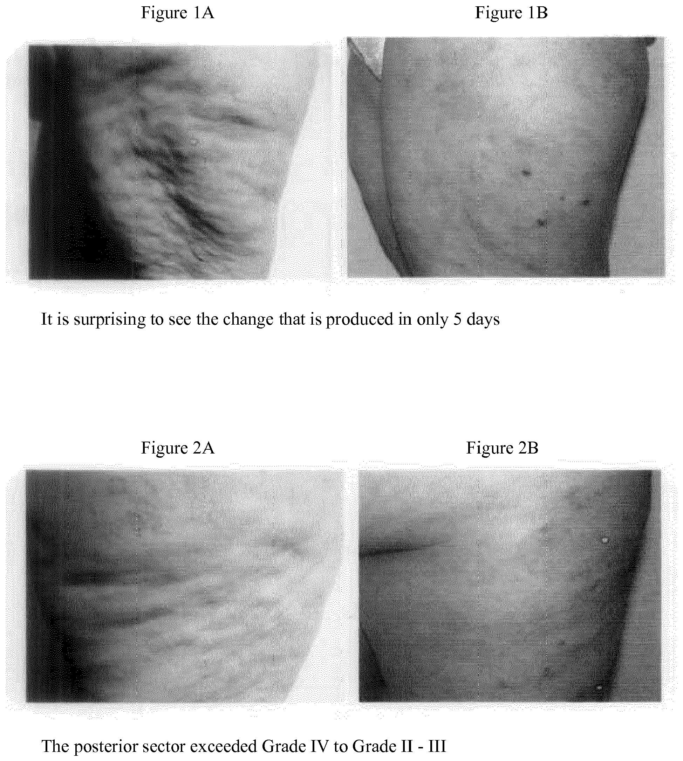

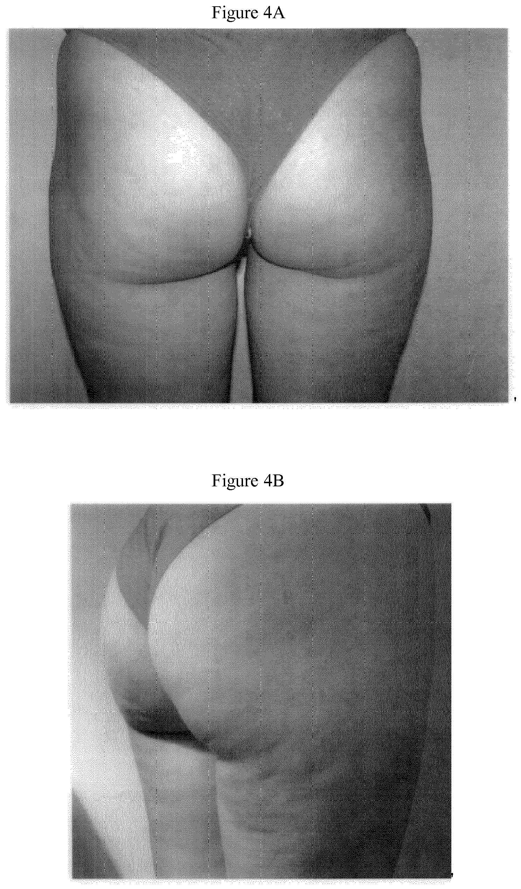

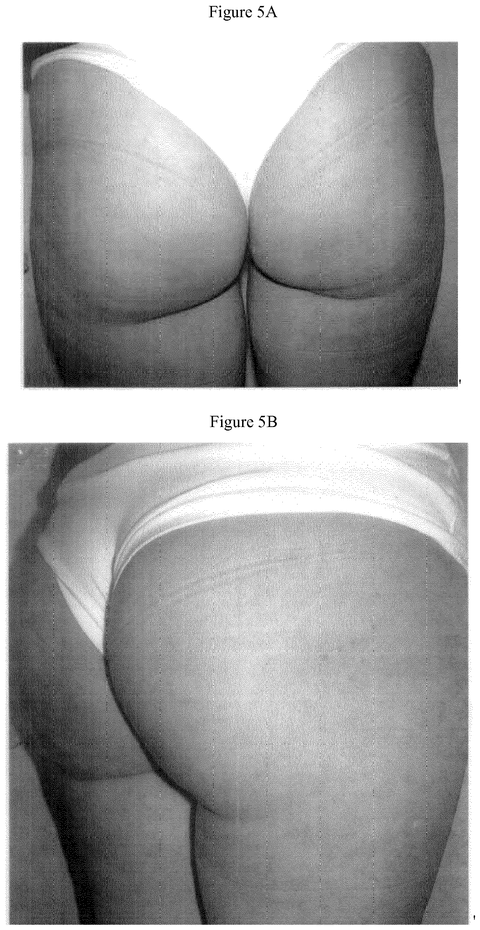

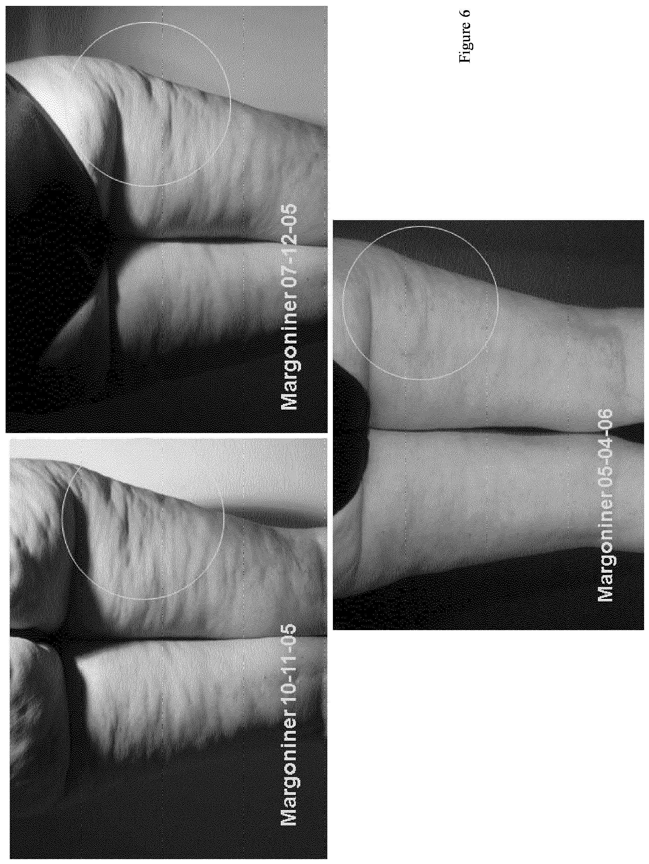

The series of photographs, **FIG. 1 to 6****,** present dramatic evidence of the success of the present invention in substantially reducing the effects and appearance of cellulites in three patients after only 1 or several treatments and only a few days after the treatments.

When the photosensitizer Temoporfin is activated with light from a diode laser, e.g., 652 nm wavelength, this produces an intracellular oxidation that is believed to act to modify the cell membrane's properties, the cytoplasm, ribosomas, Golgi's appliance, and nucleus, eventually triggering a series of events that result with the cell apostosis.

The cell when exposed to the effects of Temoporfin or other photosensitizer begins a series of morphologic changes. The plasma membrane alters and the characteristic blebbing appears. The cell volume decreases considerably and the cytoplasm condenses. The nucleus becomes smaller and chromatin become denser and eventually collapses splitting into several spheres of material.

At the end of apostosis, the cell is ingested by phagocytosis or by nearby cells avoiding the inflammatory response typical during normal cell necrosis. Even though the cell disappears, there is an increase of the collagen web. This improves the support of collagen, realignment of the collagen fibers and elastin, decreases the gelatinous consistency of the fundamental inter-cell substance, improves oxygenation and cell nutrition, and decreases toxic metabolites' retention and the edema.

Temoporfin is a very efficient generator of active oxygen which does not require a large dose of the drug nor a long exposure to light.

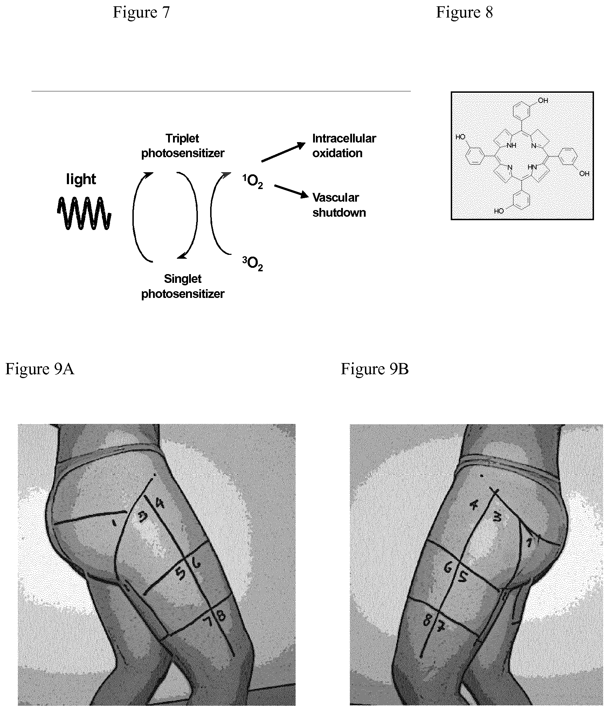

Figure 7 illustrates the process of Temoporfin's interaction upon activation.

The intracellular oxidation is responsible for the alterations of the membrane's surface and the nuclear, the mitocondrias, Golgi's appliance, the net endoplasmatic and the ribosomas resulting in the death of the cells or cell apoptosis.

Temoporfin in the past has been used in the treatment of some head and neck cancers.

Temoporfin is totally innocuous and inactive in the dark and is activated with low intensities of light and it turns into a powerful isolated-oxygen generator.

### EXAMPLE 1

A patient having cellulites is treated with Temoporfin in several sessions.

The variables in this treatment program are as follows:
1.- A determination of the grade of cellulites in the patient.
2.- A determination of the dose and concentration of Temoporfin and to confirm that it produces an improvement on the cellulites, and to further insure that it is possible to apply it directly by Mesotherapy without the need of systemic introduction.
3.- A determination as to the potency of Temoporfin based on the time of application to the time of laser application; and
4.- A determination as to accompanying treatments.

The dilutions used for treatment are presented in the following Table 1:

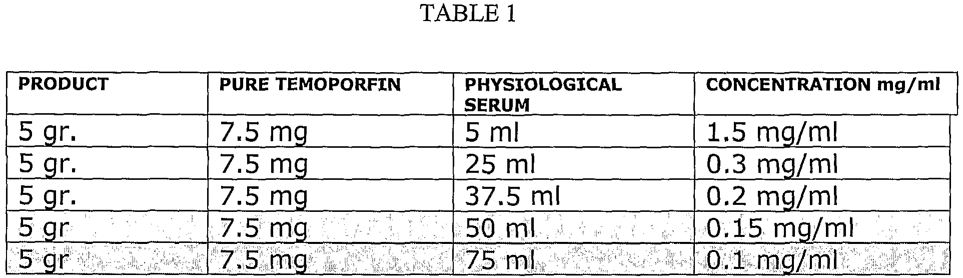

To treat cellulites a concentration of 0.005 mg/ml is applied directly by Mesotherapy. This concentration results from the study of optimal concentrations for the use of m-THPC on the treatment of tumors: 0.1 to 0.3 micrograms per gram of tumor tissue. The aesthetic doses are 10 times lower as the objective is to develop a collagen matrix and not destroy a tumor. Therefore, the aesthetic doses range from 5 to 15 µg/ 50 gr of cellulites tissues.

To apply Mesotherapy on 50 gr of tissue, 2 cc of solution is applied (area of 100 cm² with a diffusion of 0.5 cm depth).

Finally, 2 cc of Mesotherapy solution must contain 0.005 to 0.015 mg of m-THPC so its concentration must be as follows: Concentration of M-THPC for Mesotherapy on cellulites: 0.005 mg/ml to 0.01 mg/ml.

### General Procedure:

In order to determine the stage as well as the location of the cellulites in the patient, CONTACT THERMOGRAPHY may be used.

High Resolution Contact Thermography is ideal to accomplish classification of the grade of cellulites and patient tracking during treatment since small variations are evidenced during treatment.

This process measures the superficial temperature of the skin surface when placed in contact therewith by means of a plate of capsulated liquid crystals. The color of the crystals is an indication of the temperature of the underlying skin. Several plates are available having different temperature ranges appropriate for skin application. Two of these plates are shown below:

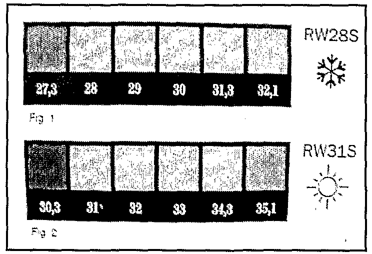

The brown colors mark hypothermic zones with little circulation and the blue colors indicate hypothermic zones with increased circulation. The temperatures are registered on the skin and provide 3 or 4 grades of temperatures of the hypodermis. The homogeneous imagery indicates the condition of the cellulites and provides exactness of diagnosis, topography and the cellulites stage. The following are examples of imagery from cellulites areas as well as the stages associated with that cellulites:

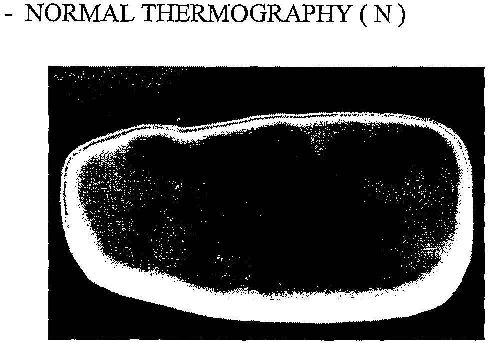

This is a uniform, unstained color image that is obtained at sectors with absence of cellulites. A reference termography may be done on the arm or on the forearm zones that in general do not present cellulites.

Evolutionary grades or cellulites stages used in the treatment of patients:

The following conditions are indicative of Stages I and II: Slowing down of the venous and lymphatic circulation; with the dilatation of the little veins of the deep cape of the dermis. Interstitial edema at zones surround the adipose cells. The exudation increases rapidly for the serum from the capillaries of the subcutaneous tissue. The zone with edema compresses the conjunctive fibers as well as the nervous elements, and it can manifest itself with pain by touch and at times spontaneous. There may be alterations of sensibility and formation of stretch marks.

There is histology of hyperplatia and hypertrophy of the reticular fibers that surround the adipose cells and the capillaries. These phases are reversible.

Thermography in these stages presents hyperthermic big and diffuse borders, surrounded stains evidence hypodermic diffuse zones and the image translates the alteration and instability of the microcirculation of the zone.

### Stages I and II (primarily II)

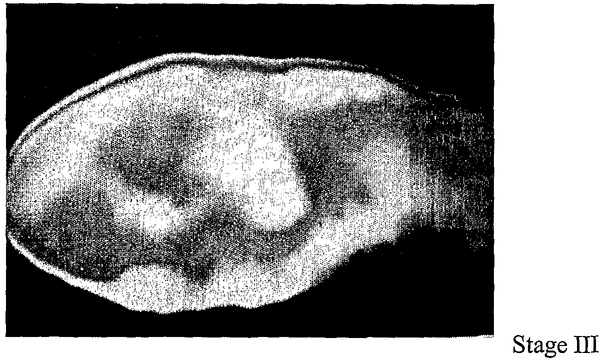

In Stage III, there is fibrous proliferation. The fibers swell up and there is a decrease of collagen, an increase in the fibrin and loss of the individual characteristics of little fibers that mask a named fibrinoide type. Collagen is deconstructed and degenerated, forming irregular, amorphous blocks, losing its structure and provoking confinement of the full adipositos of tri-glycerides. The clinical and visual aspect is termed the "orange skin." There is slowing down of cell vascular interchanges thus forming micronodules. The reversibility of the effect becomes more difficult in this phase.

Thermography evidence: The image is a multi-colored image where the predominate colors are celestial green and pink. Leopard's aspect of the skin localizes the irregularities of temperature in the skin for effect of the micronodules. The very cold zones that the black announcers begin to appear as "Black Holes" which characterize stage IV cellulite.

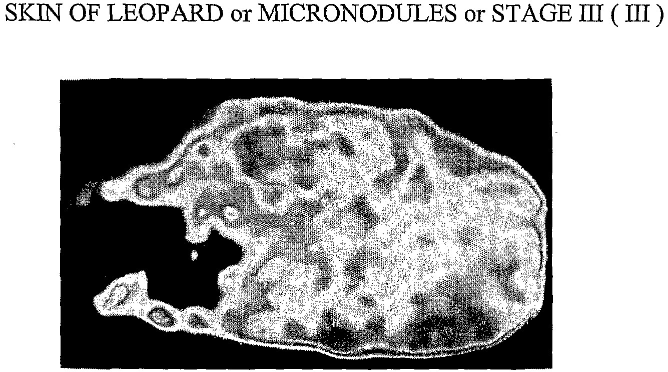

Stage IV: The fibrosis increases and compresses veins and nerves producing an alteration of the elements of the conjunctive tissue. The adipose tissue is divided into compartments like tablets with large hypothermic blocks because of its low sanguine circulation. The adipocytes are normal, but they are compressed. Several contiguous intervening micronodules fuse as one capsule making a macronodule that is palpable.

Clinically the skin presents a padded appearance with presence of painful isolated macronodules or in conglomerate forming hard plates evidences itself. In this stage there is flabbiness.

Thermographically, the image presents one of Black Holes with extensive hypodermic zones that indicate that macronodules are presence.

In this EXAMPLE, the patient is administered the Temoporfin solution as follows: In all sequences, the treatment consists 1 session only per area to be treated, and, if necessary, it is repeated in 30 days. In general, it was observed that only 1 session followed by appropriate physiotherapy, a complementary session (like ultrasound, lymph-drainage, isotonic and isometric electro-stimulation), was usually sufficient in more than 80% of the patients. If it was necessary to repeat it, an evaluation is done after 30 days.
Other treatments included in this process might include the following, as examples:
Ultrasound; Lymphodrain and presotherapy; Thermotherapy; and Isometric Electrotonotherapy.

Table 2 presents further information as to the stage of cellulite, concentration of mTHPC (Temoporfin), dose, surface area being treated; strength, power level and time of laser radiation application.

**TABLE 2**

| The PDT's application TEMOPORFIN will follow the following parameters | | | | | | |
|---|---|---|---|---|---|---|
| **CELLULITIS** | **M** - **THPC** | **DOSE** | **SURFACE** | **LATENCY** | **Watts** | **TIME PDT** |
| I HARROW I II | 0.1 mg/ml | 10 cc | 600 cm2 (20 x 30) | 30 ' | 2 W | 2 ' each 100 cm2 |
| I HARROW III | 0.2 mg/ml | 10 cc | 600 cm2 (20 x 30) | 30 ' | 2 W | 2 ' each 100 cm2 |
| I HARROW IV | 0.2 mg/ml | 10 cc | 400 cm2 (20 x 20) | 30 ' | 2.5 W | 3 ' each 100 cm2 |

These parameters are adjusted according to the patient's condition.

The following is a listing of the sequence of events in each session:
Accomplish a mapping with sectorial (See Figure 9) thermographic images and the recording of this information as to each patient for reference during treatment;
Identify zones on the patient's body where there are areas of treatment;
Disinfect skin with alcohol;
Apply the Temoporfin solution in accordance with the treatment plan established;
Apply ultrasound at the rate of 1 minute for each 100 cm² of surface which further helps distribute the product within the cellulite tissue;
Wait for 30 minutes;
Apply the laser radiation from about 6.25 to 2.5 cm from the dermic surface, with a spot of 3 cm, with a continuous mode, with a potency of 2 watts, for 2 or 3 minutes on each 100 cm² depending on grade and applied dose.

Apply a cold gel covered by plastic film to the areas of treatment;
Apply the cold gel film for 2 hours;
Allow the patient to remove the cold gel with a natural sponge applied in circles after the treatment sessions.

The results of the treatment should be followed by thermography noting cold zones, micro-nodules and black holes. The patient's body should be divided into sectors with the stage level noted for each. This is beneficial for followup treatments and consultation. Pictures of a body are included noting the different sector numbers.
Sector 1: Gluteus
Sector 2: Posterior of thigh
Sectors 3 to the 8: Anterior of thigh

Because of the low dose concentration of Temoporfin, the cost should be low so that it will be affordable.

Further, Lipofundin MCT 10 % may be applied in the tissue with there is cellulites to improvement the Temoporfin's diffusion. Further, Lipofundin may be used to dilute the Temoporfin.

### EXAMPLE 2

Temoporfin is a photosensitizer from a group that have affinity to phospholipids and cell membranes but are not soluble in triglycerides.

The appearance of cellulites, in the more severe stages, is a bubbling affect having depressed areas and hill like areas. These areas may require special treatment as follows:
1) For filling depressed areas:
   a. the diluted Temoporfin (formulated in a liposome) is mixed 0.3 mg per ml;
   b. fat from liposuction is washed and mixed with the diluted temoporfin at a ratio 5 parts to 1 part;
   c. an 18G needle is used to give the shot of this mixture in the depressed area;
   d. 30 minutes wait after shot;
   e. PDT treatment; and
   f. the treated area is washed.
2) For elevated zones or hills
   a. a mesotherapy with Temoporfin (formulated in a liposome) having 0.3 mg per ml was diluted.
   b. inject this mixture into the area of treatment;
   c. wait 30 minutes; and
   d. PDT treatment.
In general the procedure is as follows: 1. analysis and marking off of areas to be treated; 2. adding fluid to areas, after local anesthetic applied; 3. lasing of tissue within each area of treatment; 4. squeeze and suction of melted fat; and 5. application of ointments, etc., after treatments.

### EXAMPLE 3

Steps for Patient Assessment and treatment for localized cellulite:
1) Diagnostic: Clinical evaluation with anthropometric measurements, weight and pictures complemented by the use of Thermograph charts to confirm the clinical assessment of the cellulites stage (I, II, III and IV)
2) Treatment:
   a. Temoporfin (formulated in a liposome) Mesotherapy deep injection:
      1. 2 cc/100 cm² of area to be treated @ 0.05 mg/ml temoporfin dilution (Stage IV) only 1 injection used; and
      2. 2 cc/100 cm² of area to be rated @0.025 mg/ml temoporfin dilution (Stage II and III) only 1 injection;

      For example, on a buttock area of treatment, 6 or 8 cc of solution is used (@ 0.05 or 0.025 depending on the stage of the condition) For serious conditions, liposuction is used to obtain some of the patient's own fat which is cleaned and injected into the depressed areas;
   b. Lymph-drainage/soft massage: This helps to distribute the drug within the area of treatment;
   c. Wait 30 minutes;
   d. Laser Illumination: area is divided into spots to cover the whole area and a fluence of 0.8 J/cm2 is used on stage II & III (with 0.5W and time according to the area) or 1 J/cm2 on stage IV (with 0.75 W).
3) Post-Immediate:
   a. Gel application; and
   b. Light compression stockings.
4) Post @ 48 hrs: complementary sessions could be applied like: lymph-drainage, press therapy, ultrasound, gels, etc.
5) Follow-up: Assessment of results.

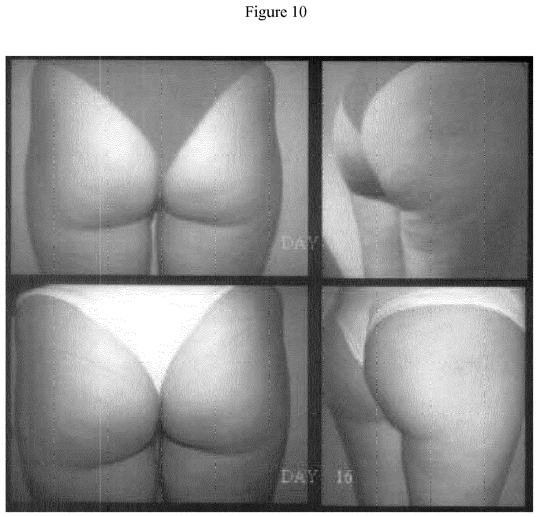

Figure 10 presents an example of patients before and after treatment.

### EXAMPLE 4

### LED Based PDT for cellulite reduction

Before starting the treatment, patient clinical history is studied and physical examination is performed. The cellulite is staged by clinical impression and by thermography.

Liposomal formulation of temoporfin diluted in 5%gluocuse solution is used. Diluted liposomal formulated temoporfin (1part) is mixed with lipofundin (9part) which helps better drug diffusion into the cellulite.

Pistol like device with trigger is used for injecting the drug into the treatment site. Single shot using the device delivers 12µl of liposomal formulated temoporfin; each injection site receives two such shots to a depth of 0.5cm. Each injection site is separated from each other by 0.5cm approximately. Dose of the drug can be increased based on the cellulite stage which needs to be treated.

Illumination is performed using LED arrays (area of the array is 4*4 cm) with wavelength 652 nm, power density 180mW/cm².

Treatment schedule: 4 LED treatments are performed in the patient. The first starts 48h after injection of the liposomal formulated temoporfin. The second, third and fourth are performed on 7, 14 and 21 days after injection respectively.

Patient is examined at the end of 21 days and later follows ups showed good improvement with better skin texture resulting in smoother skin.

For the sake of this invention and particularly for the claims, some precursors of photosensitizers become photosensitizers by the natural activity of the animal injected with them. These are considered, therefore to be included within the general word, "photosensitizers" used in the claims and general specification, as was noted earlier.

Having described preferred embodiments of the invention with reference to the accompanying drawing, it is to be understood that the invention is not limited to the precise embodiments, and that various changes and modifications may be effected therein by skilled in the art without departing from the scope of the invention as defined in the appended claims

## Claims

1. A method of reducing unwanted cellulite in selective areas of an organism comprising the steps of:
a. identifying said selective areas to be treated;
b. administering at least one photosensitizer to said organism;
c. allowing a sufficient time for said photosensitizer to accumulate in cellulite of the selective area of treatment; and
d. selectively activating by light said photosensitizer in said selective areas to reduce/eliminate cellulite
further including before step b, a step of mixing said photosensitizer into a mixture of cellular tissues from said organism,
and wherein said photosensitizer is Temoporfin (mTHPC).

2. The method of reducing unwanted cellulite according to claim 1,
wherein said cellular tissues are selected from the group consisting of adipose cells, and collagen.

3. The method of reducing unwanted cellulite according to claim 1,
wherein a treatment fluid comprising said photosensitizer and said cellular tissues are mixed in a ratio of about 1 part to 5 parts by volume.

4. The method of reducing unwanted cellulite according to claim 3,
wherein a volume of said treatment fluid administered to said selective area is adjusted according to whether said cellulite is in a depressed area of the skin or an elevated area on the skin.

5. The method of reducing unwanted cellulite according to claim 1,
wherein said administering step is by local application to the areas to be treated.

6. The method of reducing unwanted cellulite according to claim 1,
wherein said selectively activating is by means of electromagnetic radiation, wherein said electromagnetic radiation includes a wavelength matching at least one peak within said photosensitizer's absorption spectrum, and wherein said electromagnetic radiation is emitted by a light source selected from the group consisting of diode lasers, LEDs, and one or more lamps with filters.

7. The method of reducing unwanted cellulite according to claim 1,
wherein said identifying step comprises the use of thermography to determine the stage of cellulites.

8. A photosensitizer mixture for treating cellulite tissue comprising: a hydrophobic photosensitizer; and a carrier wherein
- said photosensitizer is Temoporfin (mTHPC), and
- the mixture further comprises one or more materials selected from the group consisting of hyaluronic acid, collagen, adipose cells previously removed by liposuction.

9. The photosensitizer mixture according to claim 8,
wherein said Temoporfin is present within liposomes.

10. The photosensitizer mixture according to claim 9,
wherein the selected material is fat from liposuction and wherein the fat and the Temoporfin are mixed in a ratio of 5 parts to 1 part by volume, respectively.

## Patentansprüche

1. Verfahren zur Schwächung unerwünschter Cellulite in ausgewählten Bereichen eines Organismus mit den Verfahrensschritten:
a. die zu behandelnden ausgewählten Bereiche werden identifiziert;
b. dem Organismus wird wenigstens ein Photosensibilisator verabreicht;
c. ausreichend Zeit wird gewährt, damit sich der Photosensibilisator in der Cellulite des für die Behandlung ausgewählten Bereichs anreichert; und
d. in dem ausgewählten Bereich wird der Photosensibilisator gezielt durch Licht aktiviert, um die Cellulite zu schwächen/zu entfernen,
das ferner vor Schritt b einen Schritt aufweist, in dem der Photosensibilisator in eine Mischung von Zellgeweben aus dem Organismus gemischt wird,
und bei dem der Photosensibilisator Temoporfin (mTHPC) ist.

2. Verfahren zur Schwächung unerwünschter Cellulite nach Anspruch 1,
bei dem die Zellgewebe aus der Gruppe ausgewählt sind, die aus Fettzellen und Kollagen ausgewählt sind.

3. Verfahren zur Schwächung unerwünschter Cellulite nach Anspruch 1,
bei dem ein Behandlungsfluid, das den Photosensibilisator und die Zellgewebe aufweist, in einem Volumenverhältnis von ungefähr 1 Teil zu 5 Teilen gemischt wird.

4. Verfahren zur Schwächung unerwünschter Cellulite nach Anspruch 3,
bei dem ein Volumen des Behandlungsfluids, das an dem ausgewählten Bereich verabreicht wird, daraufhin eingestellt wird, ob sich die Cellulite in einem abgesenkten Bereich der Haut befindet oder ein erhöhter Bereich auf der Haut ist.

5. Verfahren zur Schwächung unerwünschter Cellulite nach Anspruch 1,
bei dem der Verabreichungsschritt durch lokale Verabreichung an den zu behandelnden Bereichen stattfindet.

6. Verfahren zur Schwächung unerwünschter Cellulite nach Anspruch 1,
bei dem die gezielte Aktivierung mittels elektromagnetischer Strahlung stattfindet, wobei die elektromagnetische Strahlung eine Wellenlängenübereinstimmung mit einem Höchstwert innerhalb des Absorptionsspektrums des Photosensibilisators umfasst und wobei die elektromagnetische Strahlung von einer Lichtquelle emittiert ist, die aus der Gruppe ausgewählt ist, die aus Diodenlasern, LEDs und einem oder mehreren Lampen mit Filtern besteht.

7. Verfahren zur Schwächung unerwünschter Cellulite nach Anspruch 1,
bei dem der Identifikationsschritt die Verwendung von Thermographie umfasst, um das Stadium der Cellulite zu erfassen.

8. Photosensibilisatormischung zur Behandlung von Cellulitegewebe mit: einem hydrophoben Photosensibilisator und einem Träger, wobei
- der Photosensibilisator Temoporfin (mTHPC) ist, und
- die Mischung ferner ein oder mehrere Materialien umfasst, die aus der Gruppe ausgewählt sind, die aus Hyaluronsäure, Collagen oder Fettzellen ausgewählt sind, die zuvor durch Fettabsaugen entfernt worden sind.

9. Photosensibilisatormischung nach Anspruch 8,
bei der das Temoporfin innerhalb von Liposomen vorliegt.

10. Photosensibilisatormischung nach Anspruch 9,
bei der das ausgewählte Material Fett aus einer Fettabsaugung ist und bei der das Fett und Temoporfin jeweils in einem Volumenverhältnis von 5 Teilen zu 1 Teil gemischt sind.

## Revendications

1. Un procédé de réduction de cellulite indésirable dans des zones sélectionnées d'un organisme comprenant les opérations suivantes :
a. l'identification desdites zones sélectionnées à traiter,
b. l'administration d'au moins un photosensibilisateur audit organisme,
c. l'attribution d'un temps suffisant de façon à permettre audit photosensibilisateur de s'accumuler dans la cellulite de la zone de traitement sélectionnée, et
d. l'activation sélective par de la lumière dudit photosensibilisateur dans lesdites zones sélectionnées de façon à réduire/éliminer la cellulite,
comprenant en outre avant l'opération b, une opération de mélange dudit photosensibilisateur dans un mélange de tissus cellulaires provenant dudit organisme, et dans lequel ledit photosensibilisateur est Témoporfine (mTHPC).

2. Le procédé de réduction de cellulite indésirable selon la Revendication 1, dans lequel lesdits tissus cellulaires sont sélectionnés dans le groupe se composant de cellules adipeuses et de collagène.

3. Le procédé de réduction de cellulite indésirable selon la Revendication 1, dans lequel un fluide de traitement contenant ledit photosensibilisateur et lesdits tissus cellulaires est mélangé dans un rapport d'environ de 1 part sur 5 parts par volume.

4. Le procédé de réduction de cellulite indésirable selon la Revendication 3, dans lequel un volume dudit fluide de traitement administré à ladite zone sélectionnée est ajusté selon que ladite cellulite se trouve dans une zone en creux de la peau ou est une zone en relief sur la peau.

5. Le procédé de réduction de cellulite indésirable selon la Revendication 1, dans lequel ladite opération d'administration s'effectue par application locale aux zones à traiter.

6. Le procédé de réduction de cellulite indésirable selon la Revendication 1, dans lequel ladite activation sélective s'effectue au moyen d'un rayonnement électromagnétique, dans lequel ledit rayonnement électromagnétique comprend une longueur d'onde correspondant à au moins un pic à l'intérieur dudit spectre d'absorption du photosensibilisateur, et dans lequel ledit rayonnement électromagnétique est émis par une source lumineuse sélectionnée dans le groupe se composant de lasers à diode, LED et d'une ou de plusieurs lampes avec filtres.

7. Le procédé de réduction de cellulite indésirable selon la Revendication 1, dans lequel ladite opération d'identification comprend l'utilisation d'une thermographie de façon à déterminer le stade de cellulites.

8. Un mélange photosensibilisateur destiné au traitement de tissus de cellulite comprenant : un photosensibilisateur hydrophobe et un vecteur, dans lequel
- ledit photosensibilisateur est Témoporfine (mTHPC), et
- le mélange contient en outre un ou plusieurs matériaux sélectionnés dans le groupe se composant d'acide hyaluronique, de collagène, de cellules adipeuses antérieurement supprimées par liposuccion.

9. Le mélange photosensibilisateur selon la Revendication 8,
dans lequel ladite Témoporfine est présente à l'intérieur de liposomes.

10. Le mélange photosensibilisateur selon la Revendication 9,
dans lequel le matériau sélectionné est du gras provenant de la liposuccion et dans lequel le gras et la Témoporfine sont mélangés dans un rapport de 5 parts sur 1 part par volume, respectivement.
